# EUROPEAN PATENT APPLICATION

(11) **EP 4 752 896 A1**
(43) Date of publication of application: **03.06.2026**
(21) Application number: 25217164.0
(22) Date of filing: 20.11.2025
(51) Int. Cl.: G16H 20/10, G16H 50/70, G16H 70/20, G16H 70/40

(54) **METHODS AND SYSTEMS FOR PREDICTING EXPLAINABLE DRUG DOSAGE OF A SUBJECT**

(30) Priority: 27.11.2024 IN 202421092861
(71) Applicant: Tata Consultancy Services Limited, Maharashtra (IN)
(72) Inventor: MISRA, Amit Kumar, 201301 Noida, Uttar Pradesh (IN); GOEL, Anju, 110001 New Delhi (IN); SAXENA, Swanshi, 110001 New Delhi (IN); SAXENA, Amit, 201301 Noida, Uttar Pradesh (IN); KAUSHAL, Manik, 110001 New Delhi (IN); MALHOTRA, Surbhi, 110001 New Delhi (IN)
(74) Representative: Goddar, Heinz J.

(57) **Abstract**

The disclosure relates generally to methods and systems for predicting explainable drug dosage of a subject. Conventional techniques for advising drug dosage are mostly manual processes and the drug dosage advised by physician may not be effective as such dosages are not validated. The present disclosure solves the technical problems in the art with the methods and systems for predicting explainable drug dosage of the subject. According to the present disclosure, current clinical data and historical data are received from a patient repository. Drug dosage possibilities are generated based on the historical information available in the art. The possibilities and the scenarios are generated and evaluated using a possibility evaluation engine. The possibility score of each drug dosage and a weightage for each treatment decision scenario is computed. An explainability for each of the ranked possibilities is then generated to predict the explainable drug dosage of the subject.

## Description

### CROSS-REFERENCE TO RELATED APPLICATIONS AND PRIORITY

The present application claims priority to Indian application no. 202421092861, filed on November 27, 2024.

### TECHNICAL FIELD

The disclosure herein generally relates to drug dose prediction, and, more particularly, to methods and systems for predicting explainable drug dosage of a subject.

### BACKGROUND

Life threating diseases such as cancer (for example, Acute lymphoblastic leukaemia, Acute myeloid leukemia, paediatric acute lymphoblastic leukaemia, non-small cell lung cancer, and ovarian cancer) are increasing day by day across the world). Patients (alternatively referred as subjects herein after) suffering from these diseases must undergo a long treatment procedure (referred as a maintenance therapy) after initial screening (for example, chemotherapy treatment). During the maintenance therapy, the patients must frequently visit a physician who advises/suggests the dosage of suitable drug at every time the patients visit. The patients may be sensitive to the drug dosages prescribed by the physician, and based on a tolerance level of patient the physician may advise the drug dosage. To track the tolerance level of the patient, the physician needs to store the patient historical data, or they must refer the previous prescriptions.

Conventional techniques for advising drug dosage for these diseases are mostly manual processes where the physician may advise the drug dosage based on the tolerance level and the patient historical data. Treating these diseases during maintenance therapy are also not standardized across clinical practices due to variations in the level (stage) of diseases and complexities in the treatment process. The drug dosage advised by the physician may not be effective as such dosages are not validated in accordance with the tolerance level of the patient to cure in an expected time period. Furthermore, the physician may consume more time in referring to the patient historical data and in the absence of same, the drug dosage suggested by the physician may pose adverse effects in the patients.

### SUMMARY

Embodiments of the present disclosure present technological improvements as solutions to one or more of the above-mentioned technical problems recognized by the inventors in conventional systems.

In an aspect, a processor-implemented method for predicting explainable drug dosage of a subject is provided. The method including the steps of: receiving (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject; generating one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters; predicting a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models; generating a drug dosage explainability for each of the one or more dosage functions, based on a ranking of the dosing function score for each of the dosing functions, using the one or more LLMs, to predict the explainable drug dosage of the subject; evaluating an assessment for each of the one or more dosing functions, based on the drug dosage explainability generated, wherein the assessment is one of: (i) acceptance and (ii) rejection; adding each of the one or more dosing functions to one or more recommended dosing functions if the assessment associated with each of the one or more dosing functions received as accepted; validating each of the one or more dosing functions that are rejected, to obtain one or more modified dosing functions; determining a mismatch score between (i) each of the one or more dosing functions that are rejected, and (ii) the modified dosing function associated with each of the one or more dosing functions that are rejected; and adding the one or more modified dosing functions to the one or more recommended dosing functions, using the one or more LLMs, based on the mismatch score.

In another aspect, a system for predicting explainable drug dosage of a subject is provided. The system includes: a memory storing instructions; one or more Input/Output (I/O) interfaces; and one or more hardware processors coupled to the memory via the one or more I/O interfaces, wherein the one or more hardware processors are configured by the instructions to: receive (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject; generate one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters; predict a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models; generate a drug dosage explainability for each of the one or more dosage functions, based on a ranking of the dosing function score for each of the dosing functions, using the one or more LLMs, to predict the explainable drug dosage of the subject; evaluate an assessment for each of the one or more dosing functions, based on the drug dosage explainability generated, wherein the assessment is one of: (i) acceptance and (ii) rejection; add each of the one or more dosing functions to one or more recommended dosing functions if the assessment associated with each of the one or more dosing functions received as accepted; validate each of the one or more dosing functions that are rejected, to obtain one or more modified dosing functions; determine a mismatch score between (i) each of the one or more dosing functions that are rejected, and (ii) the modified dosing function associated with each of the one or more dosing functions that are rejected; and add the one or more modified dosing functions to the one or more recommended dosing functions, using the one or more LLMs, based on the mismatch score.

In yet another aspect, there are provided one or more non-transitory machine-readable information storage mediums comprising one or more instructions which when executed by one or more hardware processors cause: receiving (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject; generating one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters; predicting a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models; generating a drug dosage explainability for each of the one or more dosage functions, based on a ranking of the dosing function score for each of the dosing functions, using the one or more LLMs, to predict the explainable drug dosage of the subject; evaluating an assessment for each of the one or more dosing functions, based on the drug dosage explainability generated, wherein the assessment is one of: (i) acceptance and (ii) rejection; adding each of the one or more dosing functions to one or more recommended dosing functions if the assessment associated with each of the one or more dosing functions received as accepted; validating each of the one or more dosing functions that are rejected, to obtain one or more modified dosing functions; determine a mismatch score between (i) each of the one or more dosing functions that are rejected, and (ii) the modified dosing function associated with each of the one or more dosing functions that are rejected; and adding the one or more modified dosing functions to the one or more recommended dosing functions, using the one or more LLMs, based on the mismatch score.

In an embodiment, the one or more dosing functions are generated by: pre-processing the one or more drug dosage literatures, the one or more clinical guideline documents, and the one or more historical training data, to obtain one or more pre-processed drug dosage literatures, one or more pre-processed clinical guideline documents, and one or more pre-processed historical training data, respectively; identifying a plurality of domain parameters from the one or more pre-processed drug dosage literatures, the one or more pre-processed clinical guideline documents, and the one or more pre-processed historical training data, using the one or more LLMs, wherein the plurality of domain parameters are attributed for a drug dosage; generating one or more comprehensive responses for each of the plurality of domain parameters, from the one or more pre-processed drug dosage literatures, the one or more pre-processed clinical guideline documents, and the one or more pre-processed historical training data, using the one or more LLMs; validating each of the one or more comprehensive responses associated with each of the plurality of domain parameters using prompt engineering and a predefined validation questionnaire, to obtain one or more validated comprehensive responses associated with each of the plurality of domain parameters; generating the one or more parametrized scenarios, from the one or more domain parameters out of the plurality of domain parameters, by employing one or more boundary conditions, using the one or more validated comprehensive responses associated with each of the plurality of domain parameters; and generating the one or more dosing functions, from the one or more parametrized scenarios.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is an exemplary block diagram of a system for predicting explainable drug dosage of a subject, in accordance with some embodiments of the present disclosure.
FIGS. 2A-2B illustrate exemplary flow diagrams of a processor-implemented method for predicting explainable drug dosage of a subject, using the system of FIG. 1, in accordance with some embodiments of the present disclosure.
FIG. 3 is a flowchart showing the steps for generating the one or more dosing functions, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Patients suffering with diseases such as cancer may be sensitive to the drug dosages prescribed by the physician, and based on a tolerance level of patient the physician may advise the drug dosage. Each patient has unique conditions and so is the treatment given to the patient. The cohort of similar patients may fall into similar dose function, but their dose values given to the patient might be different. This makes the decision complex, and another layer of complexity is added through physician's decisions based on their past experiences and beliefs. Due to this not all the dosing functions are thoroughly known/evaluated by the physician before taking final decision on the dosage during the treatment process, which is also impossible to do considering the amount of time it takes to manually run through all the dosing functions and scenarios.

The present disclosure solves the technical problems in the art with the methods and systems for predicting explainable drug dosage of a subject. According to the present disclosure, current clinical data and historical data are received from a patient repository. Drug dosage possibilities (dosing functions) are generated based on the historical information available in the art such as literatures related to drug dosage of subjects suffering with the cancer (such as acute lymphoblastic leukaemia), clinical guideline documents related to the disease. Each drug dosage possibility (dosing function) contains treatment decision scenarios (interchangeably referred as 'parameterized scenarios herein after) for deciding the drug dosage for the patient in the current visit. The possibilities and the scenarios are generated and evaluated using a possibility evaluation engine (alternatively referred as a dosing function evaluation engine herein after).

The possibility score (dosing function score) of each drug dosage and a weightage for each treatment decision scenario are computed. The possibilities (dosing functions) and the scenarios are ranked by sorting in a pre-defined order based on the respective possibilities scores and the weightages. An explainability for each of the ranked possibilities is generated by prompting large language models (LLMs), to predict the explainable drug dosage of the subject. Then, the physician directly utilizes the explainable drug dosage of the subject for taking final decision on advising the drug dosage for the patient in the current visit.

In the context of the present disclosure, the words such as 'dosing' and 'dosage' are interchangeably used which mean an amount of dosage of the drug.

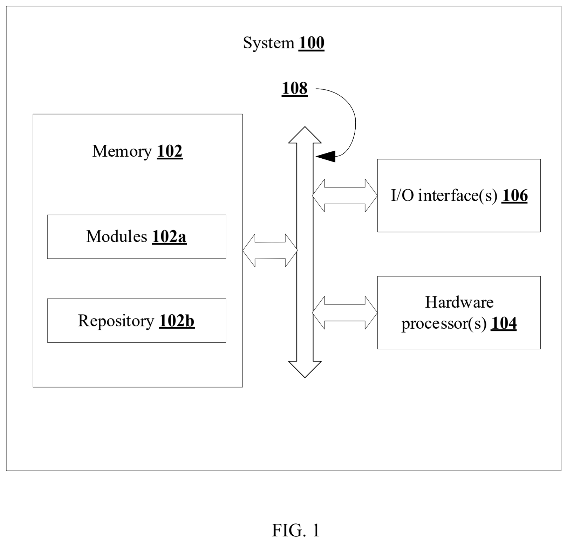

Referring now to the drawings, and more particularly to FIG. 1 through FIG. 3, where similar reference characters denote corresponding features consistently throughout the figures, there are shown preferred embodiments, and these embodiments are described in the context of the following exemplary system and/or method.

FIG. 1 is an exemplary block diagram of a system 100 for predicting explainable drug dosage of a subject, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented externally to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

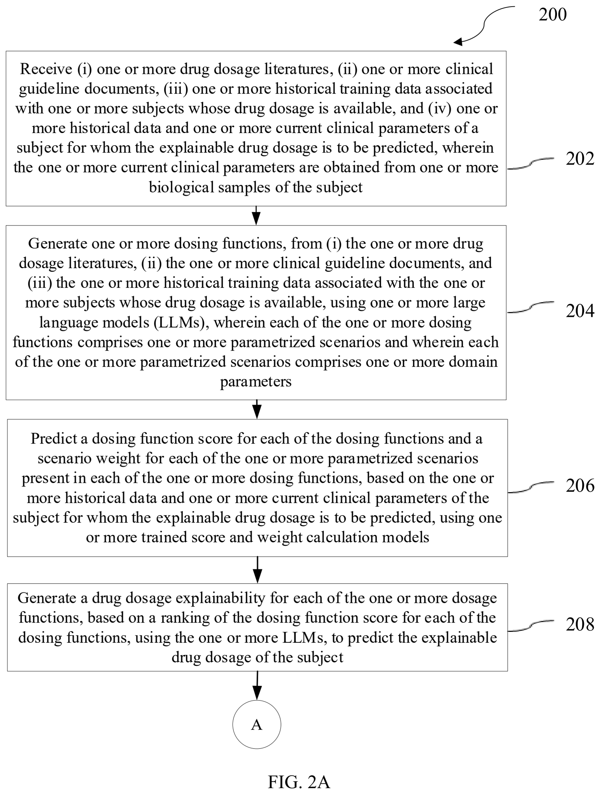

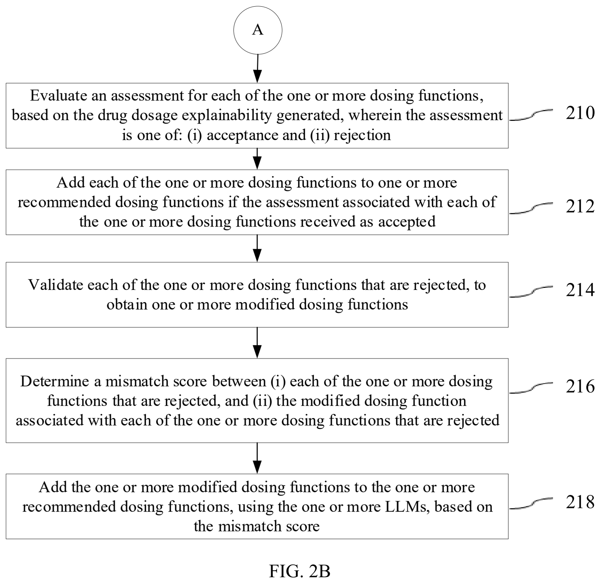

Referring to FIGS. 2A-2B, components and functionalities of the system 100 are described in accordance with an example embodiment of the present disclosure. For example, FIGS. 2A-2B illustrate exemplary flow diagrams of a processor-implemented method 200 for predicting explainable drug dosage of a subject, using the system of FIG. 1, in accordance with some embodiments of the present disclosure. Although the steps of the method 200 shown in FIGS. 2A-2B include process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the one or more hardware processors 104 of the system 100 are configured to receive one or more drug dosage literatures, one or more clinical guideline documents, and one or more historical training data associated with one or more subjects whose drug dosage is available. The one or more subjects are patients suffering with the disease and are undergoing/completed the treatment procedure (the maintenance therapy).

Further, one or more historical data and one or more current clinical parameters of a subject for whom the explainable drug dosage is to be predicted in the current visit is also received at this step. The one or more current clinical parameters are obtained from one or more biological samples of the subject. The one or more biological samples includes but are not limited to a blood sample, a urine sample, a stool sample, and a combination thereof.

At step 204 of the method 200, the one or more hardware processors 104 of the system 100 are configured to generate one or more dosing functions using one or more large language models (LLMs). The one or more dosing functions are generated from the one or more drug dosage literatures, the one or more clinical guideline documents, and the one or more historical training data associated with the one or more subjects that are received at step 202 of the method 200. In the context of the present disclosure, the one or more LLMs are obtained by either finetuning or configuring the conventional LLMs such as Gemini Pro, ChatGPT, LlAMA, and Med-LM.

Each of the one or more dosing functions (interchangeably referred as dosing possibilities and treatment possibilities) is a treatment decision or treatment possibility or a dosing possibility for deciding the drug dosage (dosage amount) for the patient in the current visit. For instance, the dosing function may be starting maintenance therapy, continuing same dose, stopping current dose, stopping the treatment completely, etc. In accordance with the present disclosure, each of the one or more dosing functions includes one or more parametrized scenarios. More specifically, different parameterized criteria and their combinations are defined for each dosing function. For instance, the different parameterized criteria are defined range criteria for blood counts range, week of visit, and so on. Each of the one or more parametrized scenarios include one or more domain parameters. In an embodiment, one or more domain parameters are the clinical parameters that are observed for a particular patient. For instance, the one or more domain parameters are defined range criteria for absolute neutrophil count (ANC), platelets (plt), hemoglobin (Hb), week of visit, and so on.

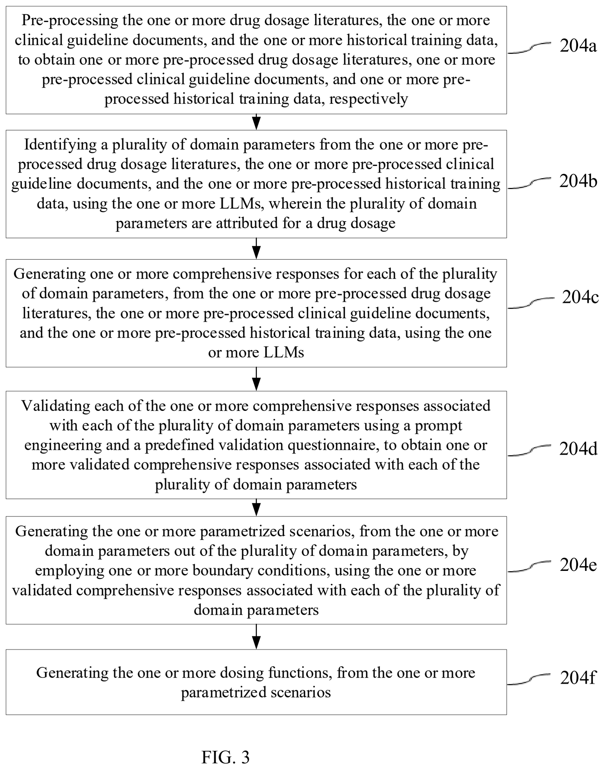

FIG. 3 is a flowchart showing the steps for generating the one or more dosing functions, in accordance with some embodiments of the present disclosure. As shown in FIG. 3, generating the one or more dosing functions is explained through steps 204a to 204f.

At step 204a, the one or more drug dosage literatures, the one or more clinical guideline documents, and the one or more historical training data, received at step 202 of the method 200 are pre-processed to obtain one or more pre-processed drug dosage literatures, one or more pre-processed clinical guideline documents, and one or more pre-processed historical training data, respectively. In an embodiment, the pre-processing includes but is not limited to, data standardization, data normalization, and outlier removal.

At step 204b, a plurality of domain parameters is identified from the one or more pre-processed drug dosage literatures, the one or more pre-processed clinical guideline documents, and the one or more pre-processed historical training data, using the one or more LLMs. The plurality of domain parameters is attributed (accounted) for a drug dosage.

At step 204c, one or more comprehensive responses are generated for each of the plurality of domain parameters generated at step 204., The one or more LLMs are used to generate the one or more comprehensive responses for each of the plurality of domain parameters, from the one or more pre-processed drug dosage literatures, the one or more pre-processed clinical guideline documents, and the one or more pre-processed historical training data.

At step 204d, each of the one or more comprehensive responses associated with each of the plurality of domain parameters are validated using prompt engineering and a predefined validation questionnaire, to obtain one or more validated comprehensive responses associated with each domain parameter. The predefined validation questionnaire includes a set of questions associated with each domain parameter, along with the actual responses. The set of questions and the actual responses associated with each domain parameter are employed by the prompt engineering in the validation process.

At step 204e, the one or more parametrized scenarios are generated, from the one or more domain parameters out of the plurality of domain parameters, by employing one or more boundary conditions, using the one or more validated comprehensive responses associated with each domain parameter obtained at step 204d during the validation process. Thus, each of the one or more parametrized scenarios includes the one or more domain parameters. Similarly, at step 204f, the one or more dosing functions are generated using the one or more parametrized scenarios. Thus, each of the one or more dosing functions include one or more parametrized scenarios.

In an embodiment, a set of dosing functions (comprising one or more dosing functions) and the underlying parametrized scenarios covering different experimental conditions, parameters, range of parameters, and expected outcomes are generated as follows:
- The initial context is used to convert it into specific small instructions (prompt) to break the domain problem into smaller parts
   o Input: curated literature, clinical guidelines
- Each instruction contains set of sequential questions (prompt) to generate comprehensive response that cover different dimensions of the knowledge for validation/correctness.
- The response of the questions within an instruction is aggregated to generate a comprehensive response for one instruction
- The response from each instruction is collectively used to generate dosing functions and their respective parameterized scenarios.
- The generated dosing functions and scenarios are transformed into a structured format.

The structured format of the dosage functions, with the parameterized scenarios are thoroughly evaluated to check using the one or more LLMs if quantitative information about parameters boundary conditions and grades are available for every scenario in each of the dosing functions. For instance, boundary condition definition for blood counts, grade of toxicity, level of side effects, etc.
- If the domain parameters for a particular scenario are quantifiable and can be expressed as definite rule, then those dosing functions and scenarios is directly stored in the information store.
   i. Scenario example: haemoglobin should be in boundary condition [x, y] for a respective dosing function
- If the domain parameters are qualitative and are not clearly defined in the scenarios, then the quantitative attributes are retrieved from the historical patient data using pre-trained machine learning (ML) models.
   i. Scenario example: Toxicity is observed. But what level of toxicity and type of toxicity could be observed based on which amount of dosage should be adjusted, is learned from the historical patient data (from the number of visits) using artificial intelligence (AI)/ML models. From historical data, the artificial intelligence (AI)/ML models are trained to extract the quantitative ranges for the qualitative parameters which is mapped to the categories/labels/classes.

In an embodiment, the AI/ML models are trained using the historical patient data as follows:
- Using different patients' data records, an AI/ML model is trained to learn different parameters' trend or range scenarios with respect to each dosing function.
- Steps: data preprocessing, data filtering, feature selection, feature engineering
   o Dosing functions are classified as labels
   o Each scenario is transformed to variables/parameters using feature engineering techniques and are then used to train ML model.
      ▪ For instance: Decision trees can help to decide a range of parameters for which a certain condition is applicable. In case of toxicity parameter, the type of toxicity is learned based on lab test information and, rate of change of blood counts, temperature, body weight and other relevant parameters can be useful in determining the grade/level of toxicity.
      ▪ Feature engineering: Scaling parameters range to attain uniformity.
- The defined parameter trends are included in the scenarios of respective dosing function and then stored in the information store.

In an embodiment, the information store stores the well-defined dosing functions and scenarios in a structured format which is later used by the system 100 as a context for the explainability.

At step 206 of the method 200, the one or more hardware processors 104 of the system 100 are configured to predict a dosing function score (alternatively referred as possibility score herein after) for each of the dosing functions and a scenario weight for each of the one or more parametrized scenarios present in each of the one or more dosing functions. The dosing function score for each of the dosing functions and the scenario weight for each of the one or more parametrized scenarios are predicted based on the one or more historical data and the one or more current clinical parameters of the subject for whom the explainable drug dosage is to be predicted.

In accordance with the present disclosure, one or more trained score and weight calculation models are employed to predict the dosing function score for each of the dosing functions and the scenario weight for each of the one or more parametrized scenarios using the one or more historical data and the one or more current clinical parameters of the subject.

In an embodiment, a dosing function evaluation engine comprising multiple computational models such as machine learning (ML) models, deep learning (DL) models, complex rule-engine, formula-based, etc., is used to predict the dosing function score for each of the dosing functions and the scenario weight for each of the one or more parametrized scenarios. The dosing function evaluation engine is trained in such a way to obtain the one or more trained score and weight calculation models which are further used for predicting the dosing function score for each of the dosing functions and the scenario weight for each of the one or more parametrized scenarios.

In an embodiment, the dosing function evaluation engine is trained as follows:
- Based on the dosing function and scenarios different model is trained on patient data fetched from patient data storage. This training is one time activity when a new model is to be added, or existing model is to be refined.
- Based on the clinical observations of current patient record and list of clinical dosing functions, the applicability of each of the computational method or combinations of methods is investigated to each clinical dosing function and its scenarios.
- Based on the selected method, the model training is performed on patient data and is used for predictions on new visit data record.
   i. Example: If parameters range and week information on the current visit lies within the range of 'start maintenance' dosing function, then one can directly apply rule-based method to support drug dosage decision. But if there is a criterion where a range of parameters lie within the scope of different dosing functions then a suitable ML/DL model is selected to evaluate the probabilities of each dosing function using different techniques.
      1. Random Forest is one of the ensemble methods that is used for multi-class classification and provides probability scores for each class which can be used to further evaluate the score of each dosing function.
      2. Random Forest also provides importance score of each parameter that can be used to allocate weightage to each scenario.
      3. Scenario weightages are combined for each dosing function and probability of that dosing function is obtained from the model. Consider these two metrics, a score is evaluated for each dosing function and stored in the patient data store for every patient record.

In an embodiment, the dosing function score for each of the dosing functions is predicted for the patient in the current visit with the trained dosing function evaluation engine is as follows:
- Patient's current visit: A patient visits the clinic, and his/her sample is collected for lab tests and clinical observations are noted. This data is stored in patient data storage as a record of that week's visit of a particular patient.
- The selected model provides the scores of each dose function for the current visit of the patient.
   ∘ Fundamental criterion for evaluating score:
      ▪ Scenarios - weightage
      ▪Dosing function - scaled combinatorial weightage -> probability
- Each dosing function and its respective score are recorded in the patient data storage.
- The dosing function and scores, and the current visit data are provided as an input to the LLM in the next step.

For example, the dosing function score for each of the dosing functions is predicted as follows based on the exemplary clinical and historical data as shown in Table 1:

**Table 1**

| Week | Cycle | ANC | Plt | Hb | Dosing function | Prescribed dosage amount of drug 6MP (6MP) | Prescribed dosage amount of drug MtX (MTX) |
|---|---|---|---|---|---|---|---|
| 8 | 1 | 2.1 | 300 | 11 | Continue Dose | 200 | 10 |
| 9 | 1 | 2.2 | 200 | 6.8 | Stop Dose | 0 | 0 |
| 10 | 1 | 4.2 | 120 | 11 | Resume LTD | 200 | 10 |
| 11 | 1 | 2.65 | 300 | 7.5 | Reduce Dose | 100 | 10 |

List of Dosing functions to be checked: continue dose, reduce dose, stop dose, dose escalation, etc.
Predicting dosing function scores for each of the dosing functions:
A) For dosing function 'reduce dose': Reduce dose implies to reduce the 100% of previous dosage given to a subject by a certain percentage/factor.
   The parametrized scenarios in the dosing function 'reduce dose':
   ➢ Scenario 1:
      ∘ Consider HB range only if the dose was previously stopped due to low Hb and If dose was stopped due to Hb in past then consider reduce dose due to Hb. AND
      ∘ ANC > 0·5 × 109/L and ANC < 0.75
      ∘ PLT > 50 × 109/L and PLT < 75 x 109/L
      ∘ HB >7 g/dL and HB < 8 g/dL
   ➢ Scenario 2:
      ∘ If just previous dose function was not "stop dose" and current blood count ranges fall in the "reduce dose" dose function. AND
      ∘ ANC > 0·5 × 109/L and ANC < 0.75
      ∘ PLT > 50 × 109/L and PLT < 75 x 109/L
      ∘ HB >7 g/dL and HB < 8 g/dL
   ➢ Scenario 3:
      ∘ Consider reduce dose if just previous dose function was reduced dose and current blood count ranges again fall in the "reduce dose" dose function.
      ∘ ANC > 0·5 × 109/L and ANC < 0.75
      ∘ PLT > 50 × 109/L and PLT < 75 x 109/L
      ∘ HB >7 g/dL and HB < 8 g/dL
   Score calculation:
      The parameters are evaluated based on the models defined in the information store
         - ANC > 0·5 × 109/L and ANC < 0.75 (if current observation of ANC is in this range: then score will be 1, else 0)
         - PLT > 50 × 109/L and PLT < 75 x 109/L (if in range: the score will be 1, else 0)
         - HB >7 g/dL and HB < 8 g/dL (if in range: the score will be 1, else 0)
      The Boolean score for each of these models is evaluated using one of the following ML models:
         1) Random Forest
         2) Logistics Regression
         3) Support vector machine (SVM)
         4) Decision Tree
         5) Extreme Gradient Boosting (XgBoost), etc.
      For the given current observation, the models evaluated score of "1" or "0" for each of the parameter.
         ANC- 0
         Hb- 1
         PLt - 0
         Hb low and stop dose parameter - 1
         Reduce dose previously parameter - 0
         Not stop dose ever parameter - 1
      Predicting scenario weights:
         Scenario 1- aggregation of parameter scores - 1
         Scenario 2- aggregation of parameters scores - 1
         Scenario 3- aggregation of parameters scores - 0.75
         Dosing function score for dosing function 'reduce dose': (1+1+0.75)/3 = 0.916 (91.6%)
B) For dosing function 'continue dose':
   The parametrized scenarios for dosing function 'continue dose':
   ➢ Scenario 1:
      ∘ Hb ≥ 8 g/dL; AND
      ∘ Neutrophil count ≥ 0.75 × 10^9/L; AND
      ∘ Platelet count ≥ 75 × 10^9/L; AND
      ∘ Cycle = 1; AND
      ∘week <8
   ➢ Scenario 2:
      ∘ Hb ≥ 8 g/dL; AND
      ∘Neutrophil count ≥ 0.75 × 10^9/L; AND
      ∘ Platelet count ≥ 75 × 10^9/L; AND
      ∘ Cycle > 1 and cycle <=8; AND
      ∘ week <6
   Score calculation:
      The Boolean score for each of these models was evaluated using one of the following ML models:
      1) Random Forest
      2) Logistics Regression
      3) SVM
      4) Decision Tree
      5) XgBoost, etc.
   For the given current observation, the models evaluated score of "1" or "0" for each of the parameter.
      ANC- 1
      Hb- 0
      PLt - 1
      Cycle =1 - 1
      Cycle>6 and <=8 -0
      Week <8- 0
      Week < 6- 0
   Weights for the scenarios:
      Scenario 1- (0+1+1+1+0)/5= 0.6
      Scenario 2- (0+1+1+0+0)/5=0.4
      The dosing function score for dosing function 'continue dose': (0.6+0.4)/2= 0.5 (50%). Likewise, the dosing function score for the dosing functions such as 'stop dose' and 'dose escalation' are predicted.

At step 208 of the method 200, the one or more hardware processors 104 of the system 100 are configured to generate a drug dosage explainability for each of the one or more dosage functions generated at step 204 of the method 200. The one or more large language models (LLMs) are configured to generate the drug dosage explainability for each of the dosing functions. In an embodiment, the one or more dosage functions generated at step 204 of the method 200 are first sorted in a pre-defined order to assign a rank for each of the one or more dosage functions. Then the one or more dosage functions that are assigned with the highest ranks (for example, top 3, or top 5, and so on) are considered for generating the drug dosage explainability using the one or more large language models (LLMs).

Finally, the drug dosage explainability generated for each of the one or more dosage functions that are assigned with the highest ranks are used for advising the explainable drug dosage of the subject in the current visit. In an embodiment, the physician may use these data including the drug dosage explainability of the one or more dosage functions and the associated ranks for advising the drug dosage of the subject in the current visit. The one or more dosage functions that are advised for every visit of the subject is stored in a repository for the future use.

In an embodiment, the drug dosage explainability for each of the one or more dosage functions is generated as follows:
• The structured set of dosing functions and scenarios are used as context to the LLM
• The dosing functions scores along with the current visit clinical observations and a particular patient's history are provided as an input to LLM.
• An explainability for every dosing function is provided as an output by LLM.
• The output is finally displayed in a well-structured format and ranked as per highest score.
• Dosing function, score, reasoning
• The score and reasoning for the dosing function with highest score is stored in the patient data storage as a recommended drug dosage criterion.

The one or more dosage functions that are considered at step 208 of the method 200 for advising the drug dosage, may not be always accurate at the first instance due to limitations in the available training data that is used for obtaining the one or more trained score and weight calculation models at step 206 of the method 200. Thus, the one or more dosage functions that are considered at step 208 of the method 200 are validated either periodically (for example, every 1 month) or after a predefined number of visits (for example, 10 visits) to get the accurate and sufficient training data so that the dosing function score and the scenario weights predicted by the one or more trained score and weight calculation models are accurate. The validation is performed as explained at steps 210 to 218 of the method 200.

At step 210 of the method 200, the one or more hardware processors 104 of the system 100 are configured to evaluate an assessment for each of the one or more dosing functions that are assigned with the highest ranks and are used for predicting the explainable drug dosage of the subject at step 208 of the method 200. At this step, the assessment is evaluated based on the drug dosage explainability generated at step 210 of the method 200. The assessment is one of: (i) acceptance and (ii) rejection. More specifically, each of the one or more dosing functions is either accepted or rejected based on a feedback obtained from the associated explainable drug dosage. The feedback is related to the justifications and reasons why each of the one or more dosing functions can be suggested to patients by the physician.

In an embodiment, the assessment for each of the one or more dosing functions is performed by one of (i) a pre-configured agent, (ii) a domain expert, and (iii) the physician. In an embodiment, the pre-configured agent is a digitized agent such as a reinforcement learning (RL) agent trained with suitable training data. The domain expert is a person who is skilled in advising the dosage amount.

At step 212 of the method 200, the one or more hardware processors 104 of the system 100 are configured to add each of the one or more dosing functions to one or more recommended dosing functions if the assessment associated with each of the one or more dosing functions received is accepted at step 210 of the method 200. The one or more recommended dosing functions are the dosing functions that can be recommended to the patients by the physician.

At step 214 of the method 200, the one or more hardware processors 104 of the system 100 are configured to validate each of the one or more dosing functions that are rejected during the assessment at step 210 of the method 200, to obtain one or more modified dosing functions. In an embodiment, the modified dosing function for each of the one or more dosing functions that are rejected, is obtained by one of (i) the pre-configured agent, (ii) the domain expert, and (iii) the physician. In an embodiment, the pre-configured agent is the digitized agent such as the reinforcement learning (RL) agent trained with the suitable training data. The domain expert is a person who is skilled in validating and advising the new dosage amount.

At step 216 of the method 200, the one or more hardware processors 104 of the system 100 are configured to determine a mismatch score between (i) each of the one or more dosing functions that are rejected at step 210 of the method 200, and (ii) the modified dosing function associated with each dosing function that is rejected which is obtained at step 214 of the method 200. More specifically, the mismatch score is determined as a difference between dosages present in each of the dosing function that is rejected and the corresponding modified dosing function.

At step 218 of the method 200, the one or more hardware processors 104 of the system 100 are configured to add each of the one or more modified dosing functions to the one or more recommended dosing functions, using the one or more large language models (LLMs), if the mismatch score is above a threshold value. The one or more recommended dosage functions of the subject are stored in a repository for future use.

In an embodiment, the steps 210 to 218 of the method 200 is executed as follows:
- Description of how an "assessment by agent" based on evidence.
- Physician either agree to the dosing function with the highest score recommended by the system 100 or disagree to it. If he agrees then the Physician decision is added as top recommended dosing function.
- But if he disagrees then the dosing function chosen by the physician along with his remarks/logic is stored in the patient data store.
- For periodic model refinement, mismatch cases (system recommendations and physician's decision) are fetched and feed it to the LLM as an input.
   i. The initial context is used to convert it into specific small instructions (prompt) to break the domain problem into smaller parts
      1. Input: mismatched records with feedback comments.
   ii. Each instruction contains set of sequential questions (prompt) to generate comprehensive response that cover different dimensions of the knowledge for validation/correctness.
   iii. The response of the questions within an instruction is aggregated to generate a comprehensive response for one instruction
   iv. The response from each instruction and provided context on scoring the significance of information extracted is collectively used to extract dosing functions and their respective parameterized scenarios from the mismatched cases.
      1. The LLM is provided with a context to look for the occurrences of each dosing function or new dosing functions in those cases and determine the logic/ reasoning behind the dosing functions and extract scenarios along with the significant score based on occurrence.
   v. The generated dosing functions and scenarios are transformed into a structured format.
- A threshold check would be applied to filter out the less significant cases based on the data.
   i. Example: least occurring cases with trivial impact on the treatment, cases where data is incomplete, mistake in the data capturing, etc.
- The generated new dosing functions (if any), scenarios are added/updated in the information store.

The methods and systems of the present disclosure generates the one or more dosing functions comprising the dosing decisions and the related domain parameters from the one or more drug dosage literatures, the one or more clinical guideline documents, and the one or more historical training data associated with the one or more subjects whose drug dosage is available. Thus, the generated dosing functions are well in standards with the requirement and accurate enough for advising the drug dosage for the subject in the current visit based on the disease type. The drug dosage explainability generated for the dosing functions by the methods and systems of the present disclosure explains the reasonings in detail. Thus, the physician can quickly decide the drug dosage accurately in less time using the drug dosage explainability.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of predicting the explainable drug dosage of the subject suffering with disease such as cancer. According to the present disclosure, current clinical data and historical data are received from a patient repository. Drug dosage possibilities are generated based on the historical information available in the art such as literatures related to drug dosage of subjects suffering with the disease, clinical guideline documents related to the disease. Each drug dosage possibility contains treatment decision scenarios for deciding the drug dosage for the patient in the current visit. The possibilities and the scenarios are generated and evaluated using a possibility evaluation engine. The possibility score of each drug dosage and a weightage for each treatment decision scenario are computed. The possibilities and the scenarios are ranked by sorting in a pre-defined order based on the respective possibilities scores and the weightages. The explainability for each of the ranked possibilities is generated by prompting large language models (LLMs), to predict the explainable drug dosage of the subject. Then, the physician directly utilizes the explainable drug dosage of the subject for taking final decision on advising the drug dosage for the patient in the current visit.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200), comprising:
receiving, via one or more input/output (I/O) interfaces, (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject (202);
generating, via one or more hardware processors, one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters (204);
predicting, via the one or more hardware processors, a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models (206); and
generating, via the one or more hardware processors, a drug dosage explainability for each of the one or more dosage functions, based on a ranking of the dosing function score for each of the dosing functions, using the one or more LLMs, to predict the explainable drug dosage of the subject (208).

2. The processor-implemented method (200) as claimed in claim 1, wherein generating the one or more dosing functions comprises:
pre-processing the one or more drug dosage literatures, the one or more clinical guideline documents, and the one or more historical training data, to obtain one or more pre-processed drug dosage literatures, one or more pre-processed clinical guideline documents, and one or more pre-processed historical training data, respectively (204a);
identifying a plurality of domain parameters from the one or more pre-processed drug dosage literatures, the one or more pre-processed clinical guideline documents, and the one or more pre-processed historical training data, using the one or more LLMs, wherein the plurality of domain parameters are attributed for a drug dosage (204b);
generating one or more comprehensive responses for each of the plurality of domain parameters, from the one or more pre-processed drug dosage literatures, the one or more pre-processed clinical guideline documents, and the one or more pre-processed historical training data, using the one or more LLMs (204c);
validating each of the one or more comprehensive responses associated with each of the plurality of domain parameters using prompt engineering and a predefined validation questionnaire, to obtain one or more validated comprehensive responses associated with each of the plurality of domain parameters (204d);
generating the one or more parametrized scenarios, from the one or more domain parameters out of the plurality of domain parameters, by employing one or more boundary conditions, using the one or more validated comprehensive responses associated with each of the plurality of domain parameters (204e); and
generating the one or more dosing functions, from the one or more parametrized scenarios (204f).

3. The processor-implemented method (200) as claimed in claim 1, further comprising:
evaluating, via the one or more hardware processors, an assessment for each of the one or more dosing functions, based on the drug dosage explainability generated, wherein the assessment is one of: (i) acceptance and (ii) rejection (210);
adding, via the one or more hardware processors, each of the one or more dosing functions to one or more recommended dosing functions if the assessment associated with each of the one or more dosing functions received as accepted (212);
validating, via the one or more hardware processors, each of the one or more dosing functions that are rejected, to obtain one or more modified dosing functions (214);
determining, via the one or more hardware processors, a mismatch score between (i) each of the one or more dosing functions that are rejected, and (ii) the modified dosing function associated with each of the one or more dosing functions that are rejected (216); and
adding, via the one or more hardware processors, the one or more modified dosing functions to the one or more recommended dosing functions, using the one or more LLMs, based on the mismatch score (218).

4. A system (100), comprising:
a memory (102) storing instructions;
one or more input/output (I/O) interfaces (106);
one or more hardware processors (104) coupled to the memory (102) via the one or more I/O interfaces (106), wherein the one or more hardware processors (104) are configured by the instructions to:
receive via the one or more I/O interfaces (106), (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject;
generate one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters;
predict a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models; and
generate a drug dosage explainability for each of the one or more dosage functions, based on a ranking of the dosing function score for each of the dosing functions, using the one or more LLMs, to predict the explainable drug dosage of the subject.

5. The system (100) as claimed in claim 4, wherein the one or more hardware processors (104) are configured to generate the one or more dosing functions, by:
pre-processing the one or more drug dosage literatures, the one or more clinical guideline documents, and the one or more historical training data, to obtain one or more pre-processed drug dosage literatures, one or more pre-processed clinical guideline documents, and one or more pre-processed historical training data, respectively;
identifying a plurality of domain parameters from the one or more pre-processed drug dosage literatures, the one or more pre-processed clinical guideline documents, and the one or more pre-processed historical training data, using the one or more LLMs, wherein the plurality of domain parameters are attributed for a drug dosage;
generating one or more comprehensive responses for each of the plurality of domain parameters, from the one or more pre-processed drug dosage literatures, the one or more pre-processed clinical guideline documents, and the one or more pre-processed historical training data, using the one or more LLMs;
validating each of the one or more comprehensive responses associated with each of the plurality of domain parameters using prompt engineering and a predefined validation questionnaire, to obtain one or more validated comprehensive responses associated with each of the plurality of domain parameters;
generating the one or more parametrized scenarios, from the one or more domain parameters out of the plurality of domain parameters, by employing one or more boundary conditions, using the one or more validated comprehensive responses associated with each of the plurality of domain parameters; and
generating the one or more dosing functions, from the one or more parametrized scenarios.

6. The system (100) as claimed in claim 4, wherein the one or more hardware processors (104) are further configured to:
evaluate an assessment for each of the one or more dosing functions, based on the drug dosage explainability generated, wherein the assessment is one of: (i) acceptance and (ii) rejection;
add each of the one or more dosing functions to one or more recommended dosing functions if the assessment associated with each of the one or more dosing functions received as accepted;
validate each of the one or more dosing functions that are rejected, to obtain one or more modified dosing functions;
determine a mismatch score between (i) each of the one or more dosing functions that are rejected, and (ii) the modified dosing function associated with each of the one or more dosing functions that are rejected; and
add the one or more modified dosing functions to the one or more recommended dosing functions, using the one or more LLMs, based on the mismatch score.

7. One or more non-transitory machine-readable information storage mediums comprising one or more instructions which when executed by one or more hardware processors cause:
receiving, (i) one or more drug dosage literatures, (ii) one or more clinical guideline documents, (iii) one or more historical training data associated with one or more subjects whose drug dosage is available, and (iv) one or more historical data and one or more current clinical parameters of a subject for whom the explainable drug dosage is to be predicted, wherein the one or more current clinical parameters are obtained from one or more biological samples of the subject;
generating one or more dosing functions, from (i) the one or more drug dosage literatures, (ii) the one or more clinical guideline documents, and (iii) the one or more historical training data associated with the one or more subjects whose drug dosage is available, using one or more large language models (LLMs), wherein each of the one or more dosing functions comprises one or more parametrized scenarios and wherein each of the one or more parametrized scenarios comprises one or more domain parameters;
predicting a dosing function score for each of the dosing functions and a scenario weight for each of the one or more parametrized scenarios present in each of the one or more dosing functions, based on the one or more historical data and one or more current clinical parameters of the subject for whom the explainable drug dosage is to be predicted, using one or more trained score and weight calculation models; and
generating a drug dosage explainability for each of the one or more dosage functions, based on a ranking of the dosing function score for each of the dosing functions, using the one or more LLMs, to predict the explainable drug dosage of the subject.

8. The one or more non-transitory machine-readable information storage mediums as claimed in claim 7, wherein generating the one or more dosing functions comprises:
pre-processing the one or more drug dosage literatures, the one or more clinical guideline documents, and the one or more historical training data, to obtain one or more pre-processed drug dosage literatures, one or more pre-processed clinical guideline documents, and one or more pre-processed historical training data, respectively;
identifying a plurality of domain parameters from the one or more pre-processed drug dosage literatures, the one or more pre-processed clinical guideline documents, and the one or more pre-processed historical training data, using the one or more LLMs, wherein the plurality of domain parameters are attributed for a drug dosage;
generating one or more comprehensive responses for each of the plurality of domain parameters, from the one or more pre-processed drug dosage literatures, the one or more pre-processed clinical guideline documents, and the one or more pre-processed historical training data, using the one or more LLMs;
validating each of the one or more comprehensive responses associated with each of the plurality of domain parameters using prompt engineering and a predefined validation questionnaire, to obtain one or more validated comprehensive responses associated with each of the plurality of domain parameters;
generating the one or more parametrized scenarios, from the one or more domain parameters out of the plurality of domain parameters, by employing one or more boundary conditions, using the one or more validated comprehensive responses associated with each of the plurality of domain parameters; and
generating the one or more dosing functions, from the one or more parametrized scenarios.

9. The one or more non-transitory machine-readable information storage mediums as claimed in claim 7, wherein the one or more instructions which when executed by the one or more hardware processors further cause:
evaluating an assessment for each of the one or more dosing functions, based on the drug dosage explainability generated, wherein the assessment is one of: (i) acceptance and (ii) rejection;
adding each of the one or more dosing functions to one or more recommended dosing functions if the assessment associated with each of the one or more dosing functions received as accepted;
validating each of the one or more dosing functions that are rejected, to obtain one or more modified dosing functions;
determining a mismatch score between (i) each of the one or more dosing functions that are rejected, and (ii) the modified dosing function associated with each of the one or more dosing functions that are rejected; and
adding the one or more modified dosing functions to the one or more recommended dosing functions, using the one or more LLMs, based on the mismatch score.
